(19) Europäisches Patentamt
European Patent Office
Office européen des brevets

(11) Veröffentlichungsnummer: **0 224 178**
**B1**

(12) # EUROPÄISCHE PATENTSCHRIFT

(45) Veröffentlichungstag der Patentschrift: **01.08.90**

(51) Int. Cl.[5]: **C07D 215/56**, C07D 405/14, C07D 409/14, A61K 31/495

(21) Anmeldenummer: **86115955.6**

(22) Anmeldetag: **18.11.86**

(54) **1-Cyclopropyl-6-fluor-1,4-dihydro-4-oxo-7-(1-piperazinyl)-3-chinolincarbonsäuren, Verfahren zu ihrer Herstellung sowie diese enthaltende antibakterielle Mittel.**

(30) Priorität: **28.11.85 DE 3542002**

(43) Veröffentlichungstag der Anmeldung:
**03.06.87 Patentblatt 87/23**

(45) Bekanntmachung des Hinweises auf die Patenterteilung:
**01.08.90 Patentblatt 90/31**

(84) Benannte Vertragsstaaten:
**AT BE CH DE ES FR GB IT LI NL SE**

(56) Entgegenhaltungen:
**EP-A- 0 049 355**
**EP-A- 0 113 092**
**DE-A- 3 142 854**

**Die Akte enthält technische Angaben, die nach dem Eingang der Anmeldung eingereicht wurden und die nicht in dieser Patentschrift enthalten sind.**

(73) Patentinhaber: **BAYER AG,**
**D-5090 Leverkusen 1 Bayerwerk(DE)**

(72) Erfinder: **Petersen, Uwe, Dr., Auf dem Forst 4, D-5090 Leverkusen 1(DE)**
Erfinder: **Grohe, Klaus, Dr., Am Wasserturm 10, D-5068 Odenthal(DE)**
Erfinder: **Zeiler, Hans-Joachim, Dr., Elsbeekerstrasse 46, D-5620 Velbert 15(DE)**
Erfinder: **Metzger, Karl Georg, Dr., Pahlkestrasse 75, D-5600 Wuppertal(DE)**

**EP 0 224 178 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue im Piperazinteil substituierte 1-Cyclopropyl-6-fluor-1,4-dihydro-4-oxo-7-(1-piperazinyl)-3-chinolincarbonsäuren, Verfahren zu ihrer Herstellung sowie diese enthaltende antibakterielle Mittel.

Es ist bekannt geworden, daß 1-Cyclopropyl-6-fluor-1,4-dihydro-4-oxo-7-(1-piperazinyl)-3-chinolincarbonsäuren als antimikrobielle Wirkstoffe verwendet werden können (Europäische Patentanmeldung 49 355 und Deutsche Patentanmeldung 3 142 854).

Es wurde nun gefunden, daß die neuen im Piperazinteil substituierten 1-Cyclopropyl-6-fluor-1,4-dihydro-4-oxo-7-(1-piperazinyl)-3-chinolincarbonsäuren der Formel (I)

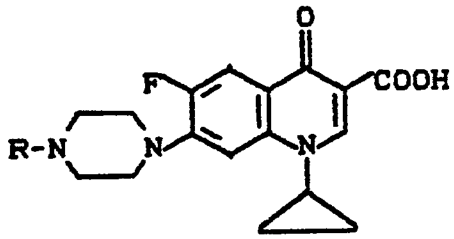

(I)

in welcher
R für verzweigtes oder unverzweigtes durch Hydroxy oder Methoxy zwei- bis dreifach substituiertes Propyl, verzweigtes oder unverzweigtes, durch Hydroxy oder Methoxy 1-bis 3-fach substituiertes Butyl und für unsubstituiertes tert.-Butyl steht, ferner für 2-Methylthioethyl, Trifluormethylthiomethyl, 2-Trifluormethylthioethyl, Cycloalkyl mit 3 bis 6 Kohlenstoffatomen, gegebenenfalls durch Hydroxy substituiertes Cycloalkenyl mit 5 oder 6 Kohlenstoffatomen, 1,1-Dioxidotetrahydrothiophen-3-yl, Cyclopropylmethyl, 1-Phenylethyl, Furylmethyl sowie für gegebenenfalls durch Phenyl substituiertes Allyl und Propargyl steht,
und deren pharmazeutisch verwendbaren Hydrate, Säureadditionssalze, Alkali-, Erdalkali-, Silber- und Guanidiniumsalze, sowie in Form ihrer Ester und in anderen üblichen Prodrug-Formen eine hohe antibakterielle Wirkung aufweisen.

Sie eignen sich daher als Wirkstoffe für die Human- und Veterinärmedizin, wobei zur Veterinärmedizin auch die Behandlung von Fischen zur Therapie oder Vorbeugung bakterieller Infektionen zu zählen ist.

Bevorzugt sind diejenigen Verbindungen der Formel (I), in denen
R für verzweigtes oder unverzweigtes durch Hydroxy oder Methoxy zwei- bis dreifach substituiertes Propyl, verzweigtes oder unverzweigtes, durch Hydroxy oder Methoxy 1-bis 3-fach substituiertes Butyl und für unsubstituiertes tert.-Butyl steht, ferner für Trifluormethylthiomethyl, 2-Trifluormethylthioethyl, Cycloalkyl mit 3 bis 6 Kohlenstoffatomen, gegebenenfalls durch Hydroxy substituiertes Cyclohexenyl, 1,1-Dioxidotetrahydrothiophen-3-yl, Cyclopropylmethyl, Furylmethyl sowie für gegebenenfalls durch Phenyl substituiertes Allyl und Propargyl steht.

Unter Prodrugs sind hier Verbindungen zu verstehen, die über die Carboxylgruppe der erfindungsgemäßen Chinolon-3-carbonsäuren einen esterartig gebundenen Rest tragen und im Körper unter Abspaltung dieses Restes wieder das Wirkstoffmolekül freisetzen. Durch eine derartige Prodrug-Bildung kann unter anderem die Bioverfügbarkeit von Wirkstoffen erhöht werden [siehe auch Pharmazie 38, 663 (1983)].

Außer den einfachen Methyl- und Ethylestern der erfindungsgemäßen Chinolon-3-carbonsäuren können auch folgende Prodrugs der Verbindungen der Formel (I) hergestellt werden, die einen üblichen Prodrug-Rest E tragen:

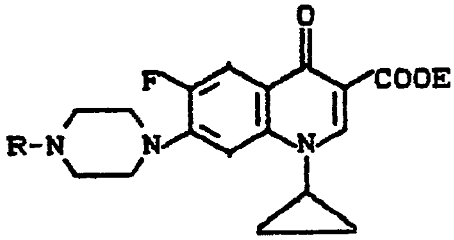

E = $-CH_2-O-CO-CH_3$, $-CH_2-O-CO-C(CH_3)_3$,

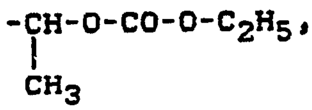, 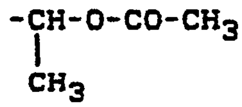

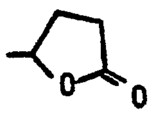, 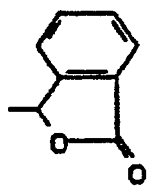, 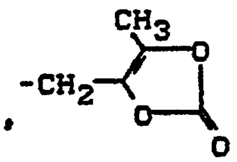, $-CH_2-CH_2-N(CH_3)_2$,

$-CH_2CH_2-N(C_2H_5)_2$, 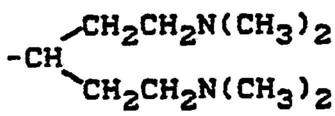, 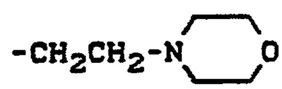

Weiterhin wurde gefunden, daß man die Verbindungen der Formel (I) erhält, wenn man eine Verbindung der Formel (II)

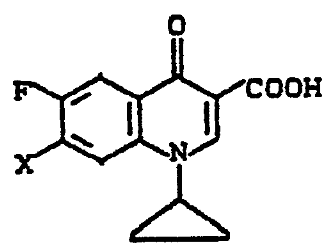

(II),

in welcher
X für Fluor oder Chlor steht,
mit Piperazinderivaten der Formel (III)

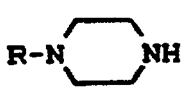

(III),

in welcher
R die oben angegebene Bedeutung hat, gegebenenfalls in Gegenwart von Säurebindern umsetzt (Methode A).

Erfindungsgemäße Verbindungen der Formel (I) können auch erhalten werden, indem man 1-Cyclopropyl-6-fluor-1,4-dihydro-4-oxo-7-(1-piperazinyl)-3-chinolincarbonsäure der Formel (IV)

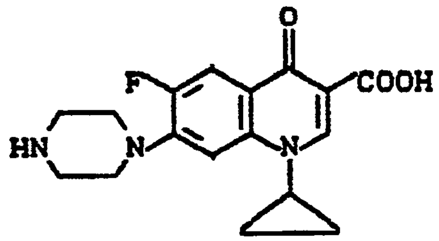

(IV)

mit Verbindungen der Formel (V)

R-Y (V)

in welcher

R die oben angegebene Bedeutung hat, jedoch nicht tert.-Butyl, Tris(hydroxymethyl)-methyl oder Cycloalkyl sein kann, und

Y Halogen, insbesondere Chlor, Brom oder Jod bedeutet,

gegebenenfalls in Gegenwart von Säurebindern umsetzt (Methode B).

Verwendet man bei der Umsetzung nach Methode A 1-Cyclopropyl-6,7-difluor-1,4-dihydro-4-oxo-3-chinolincarbonsäure und N-(tert.-Butyl)-piperazin als Ausgangsstoffe, so kann der Reaktionsablauf durch das folgende Formelschema wiedergegeben werden:

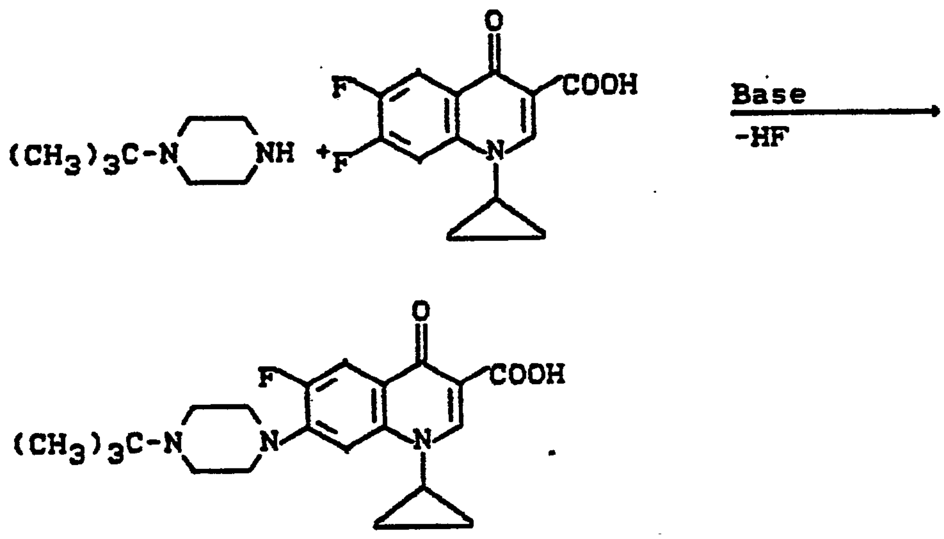


Verwendet man beispielsweise bei der Umsetzung nach Methode B 1-Cyclopropyl-6-fluor-1,4-dihydro-4-oxo-7-(1-piperazinyl)-3-chinolincarbonsäure und Allylbromid als Ausgangsstoffe, so kann der Reaktionsablauf durch das folgende Formelschema wiedergegeben werden:

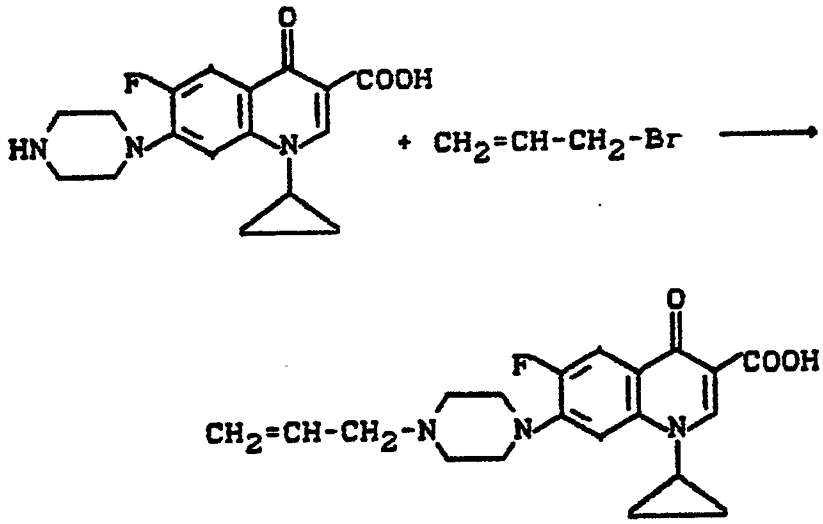

x HBr

Die als Ausgangsstoff nach Methode A verwendete 7-Chlor-1-cyclopropyl-6-fluor-1,4-dihydro-4-oxo-3-chinolincarbonsäure (Deutsche Patentanmeldung 3 142 854) und die 1-Cyclopropyl-6,7-difluor-1,4-dihydro-4-oxo-3-chinolincarbonsäure (Europäische Patentanmeldung 113 091) sind bekannt.

Die als Ausgangsstoffe nach Methode A verwendeten Piperazinderivate der Formel (III) sind zum Teil bekannt oder lassen sich nach folgendem Verfahren herstellen:

Die Umsetzung von Bis(2-hydroxyethyl)-amin (1) mit 2-Nitrophenylsulfenylchlorid (NPS-Cl) führt zum N-(2-Nitrophenylsulfenyl)-bis(2-hydroxyethyl)-amin (2), das mit 4-Toluolsulfonylchlorid zum Bissulfonat (3) umgesetzt wird. (3) läßt sich durch Erhitzen mit primären Aminen in einem Lösungsmittel wie zum Beispiel Isopropanol in Gegenwart überschüssigem Amins oder einer Hilfsbase wie Kaliumcarbonat zu den NPS-geschützten Piperazinderivaten (4) cyclisieren. Nach Abspaltung der NPS-Schutzgruppe mit 2-Mercaptobenzthiazol/Salzsäure erhält man das entsprechende Piperazinderivat als Bis-hydrochlorid (5), aus dem sich mit Alkalihydroxiden die Piperazinderivate (III) als freie Basen herstellen lassen.

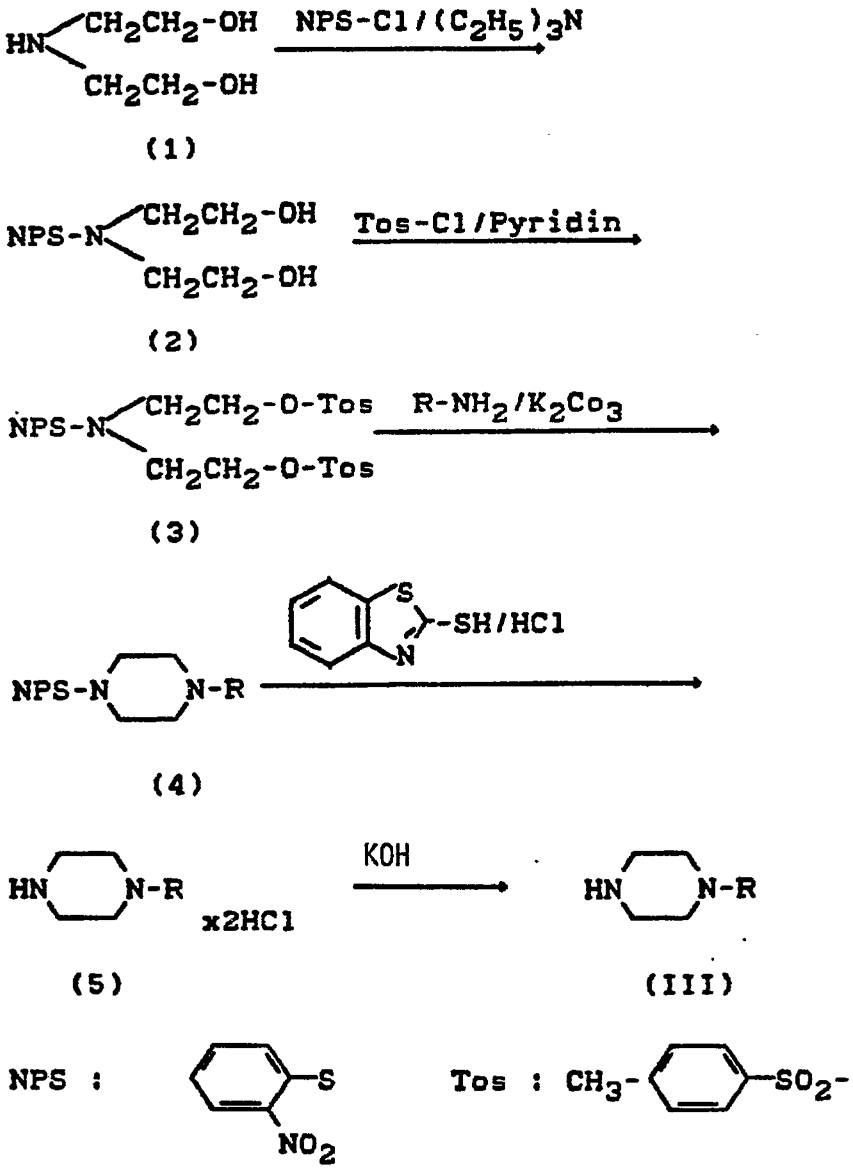


(1)

(2)

(3)

(4)

(5)

(III)

NPS :

Tos :

Als Beispiele für Verbindungen der Formel (III) seien genannt:
1-(tert.-Butyl)-piperazin, 1-[Tris(hydroxymethyl)-methyl]-piperazin, 1-(1-Phenylethyl)-piperazin, 1-Cinnamyl-piperazin, 1-Cyclopropyl-piperazin, 1-Cyclobutyl-piperazin, 1-Cyclopentyl-piperazin, 1-Cyclohexyl-piperazin, 1-(2-Furylmethyl)-piperazin, 1-(3-Furylmethyl)-piperazin, 1-(1,1-Dioxido-tetrahydrothiophen-3-yl)-piperazin.

Spezielle Piperazinderivate der Formel (III) werden hergestellt, indem man Piperazin (6) mit einem Epoxid zu einem Gemisch von Mono- und Bissubstitutionsprodukt umsetzt und das gewünschte monosubstituierte Piperazin chromatographisch abtrennt. Als Beispiel sei die Umsetzung von Piperazin mit 4,4-Dimethyl-3,5,8-trioxabicyclo[5,1,0] octan(7)(J. Org. Chem. 41, 2469 [1976]) im Formelschema aufgeführt:

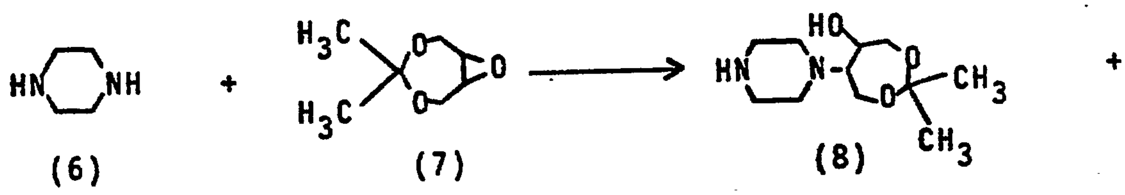

(6) (7) (8)

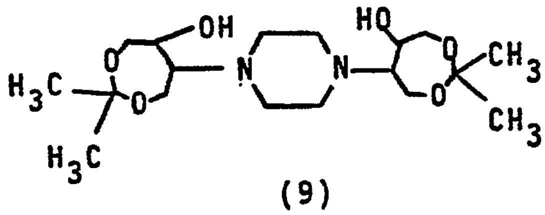

(9)

Analog erhält man mit 1,4-Cyclohexadien-monoepoxid (1-(6-Hydroxy-3-cyclohexenyl)-piperazin.

Die Hydrolyse von (8) mit verdünnter Salzsäure führt unter Abspaltung der Isopropylidengruppe zu 1-(1,3,4-Trihydroxy-2-butyl)-piperazin.

Die als Ausgangsverbindung nach Methode B verwendete 1-Cyclopropyl-6-fluor-1,4-dihyrdo-4-oxo-7-(1-piperazinyl)-3-chinolincarbonsäure der Formel (IV) ist aus der Deutschen Patentanmeldung 3 142 854 bekannt.

Die als Ausgangsverbindungen nach Methode B verwendeten Halogenverbindungen der Formel (V) sind bekannt oder lassen sich nach bekannten Methoden herstellen. Als Beispiele seien genannt: 4-Hydroxybutylchlorid, Trifluormethylthiomethylchlorid, 2-Trifluormethylthioethylbromid, Allylbromid, Propargylchlorid, Benzylbromid, Cyclopropylmethylchlorid, α-Methylbenzylbromid.

Die Umsetzung von (II) mit (III) gemäß Methode A, bei der die Piperazinderivate (III) auch in Form ihrer Hydrochloride eingesetzt werden können, wird vorzugsweise in einem Verdünnungsmittel wie Dimethylsulfoxid, N,N-Dimethylformamid, Hexamethyl-phosphorsäuretrisamid, Sulfolan, Acetonitril, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindungsmittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diaza-bicyclo-[2,2,2]-octan (DABCO), 1,8-Diazabicylo[5,4,0]undec-7-en (DBU) oder überschüssiges Amin (III).

Die Reaktionstemperaturen können in einem größeren Bereich variiert werden. Im allgemeinen arbeitet man zwischen etwa 20 und 200°C, vorzugsweise zwischen 80 und 180°C.

Die Umsetzung kann bei Normaldruck, aber auch bei erhöhtem Druck durchgeführt werden. Im allgemeinen arbeitet man bei Drucken zwischen etwa 1 und 100 bar, vorzugsweise zwischen 1 und 10 bar.

Bei der Durchführung des erfindungsgemäßen Verfahrens setzt man auf 1 Mol der Chinoloncarbonsäure (II) 1 bis 15 Mol, vorzugsweise 1 bis 6 Mol des Piperazins (III) ein.

Freie Hydroxygruppen können während der Umsetzung durch eine geeignete Hydroxyschutzgruppe, zum Beispiel durch die Isopropylidengruppe oder den Tetrahydropyranylrest, geschützt und nach Beendigung der Reaktion wieder freigesetzt werden.

Die Umsetzung von (IV) mit (V) wird vorzugsweise in einem Verdünnungsmittel wie Dimethylsulfoxid, Dioxan, N,N-Dimethylformamid, Hexamethyl-phosphorsäure-trisamid, Sulfolan, Wasser, einem Alkohol wie Methanol, Ethanol, n-Propanol, Isopropanol, Glykolmonomethylether oder Pyridin vorgenommen. Ebenso können Gemische dieser Verdünnungsmittel verwendet werden.

Als Säurebinder können alle üblichen anorganischen und organischen Säurebindemittel verwendet werden. Hierzu gehören vorzugsweise die Alkalihydroxide, Alkalicarbonate, organische Amine und Amidine. Als besonders geeignet seien im einzelnen genannt: Triethylamin, 1,4-Diazabicyclo-[2,2,2]octan (DABCO) oder 1,8-Diazabicylo[5,4,0]undec-7-en (DBU).

Zur Beschleunigung der Umsetzung können Phasentransferkatalysatoren wie Tetrabutylammoniumbromid, Tetrabutylammoniumchlorid oder Benzyl-triethyl-ammoniumchlorid zugegeben werden.

Die Reaktionstemperaturen können in einem größeren Bereich variiert werden. Im allgemeinen arbeitet man zwischen etwa 20 und etwa 180°C, vorzugsweise zwischen 40 und 110°C.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise zwischen 1 und 10 bar.

Bei der Durchführung des erfindungsgemäßen Verfahrens gemäß Methode B setzt man auf 1 Mol der Verbindung (IV) 1 bis 4 Mol, vorzugsweise 1 bis 1,5 Mol der Verbindung (V) ein.

Die Herstellung der Säureadditionssalze der erfindungsgemäßen Verbindungen erfolgt in üblicher Weise zum Beispiel durch Lösen des Betains in überschüssiger wäßriger Säure und Ausfällen des Salzes mit einem mit Wasser mischbaren organischen Lösungsmittel wie Methanol, Ethanol, Aceton, Acetonitril. Man kann auch äquivalente Mengen Betain und Säure in Wasser bis zur Lösung erhitzen und anschließend bis zur Trockne eindampfen. Als pharmazeutisch verwendbare Salze sind beispielsweise die Salze von Salzsäure, Schwefelsäure, Essigsäure, Glykolsäure, Milchsäure, Bernsteinsäure, Zitronensäure, Weinsäure, Methansulfonsäure, Galacturonsäure, Gluconsäure, Embonsäure, Glutaminsäure oder Asparaginsäure zu verstehen.

Die Alkali- oder Erdalkalisalze werden beispielsweise durch Lösen des Betains in unterschüssiger Alkali- oder Erdalkalilauge, Filtration von ungelöstem Betain und Eindampfen des Filtrats bis zur Trockne erhalten. Pharmazeutisch geeignet sind Natrium-, Kalium- oder Calciumsalze. Durch Umsetzung eines Alkali- oder Erdalkalisalzes mit einem geeigneten Silbersalz wie Silbernitrat werden die entsprechenden Silbersalze der 1,4-Dihydro-4-oxo-chinolin-3-carbonsäuren erhalten.

Außer den in den Beispielen aufgeführten Verbindungen seien als neue Wirkstoffe im einzelnen genannt:
1-Cyclopropyl-6-fluor-1,4-dihydro-4-oxo-7-[4-(1-phenylethyl)-1-piperazinyl]-3-chinolincarbonsäure,
1-Cyclopropyl-6-fluor-1,4-dihydro-7-{4-[tris(hydroxymethyl)methyl]-1-piperazinyl}-4-oxo-3-chinolincarbonsäure,
7-(4-Cyclobutyl-1-piperazinyl)-1-cyclopropyl-6-fluor-1,4-dihydro-4-oxo-3-chinolincarbonsäure,
7-(4-Cyclopentyl-1-piperazinyl)-1-cyclopropyl-6-fluor-1,4-dihydro-4-oxo-3-chinolincarbonsäure.

Beispiel für eine erfindungsgemäße Tablette

Jede Tablette enthält:

| | |
|---|---|
| Verbindung des Beispiels 1 | 583,0 mg |
| Mikrokristalline Cellulose | 55,0 mg |
| Maisstärke | 72,0 mg |
| Poly-(1-vinyl-2-pyrrolidon) unlöslich | 30,0 mg |
| Hochdisperses Siliciumdioxid | 5,0 mg |
| Magnesiumstearat | 5,0 mg |
| | 750,0 mg |

Die Lackhülle enthält:

| | |
|---|---|
| Poly-(O-hydroxypropyl-O-methyl)-cellulose 15 cp | 6,0 mg |
| Macrogol 4000 rec. INN Polyethylenglykole (DAB) | 2,0 mg |
| Titan-(IV)-oxid | 2,0 mg |
| | 10,0 mg |

Die erfindungsgemäßen Verbindungen zeigen bei geringer Toxizität ein breites antibakterielles Spektrum gegen gram-positive und gram-negative Keime, insbesondere gegen Enterobakteriaceen; vor allem auch gegen solche, die resistent sind gegen verschiedene Antibiotika, wie z.B. Penicilline, Cephalosphorine, Aminoglykoside, Sulfonamide, Tetracycline.

Diese wertvollen Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z.B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gram-negative und gram-positive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und

systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:
Gram-positive Kokken, z.B. Staphylokokken (Staph. aureus, Staph. epidermidis) und Streptokokken (Strept. agalactiae, Strept. faecalis, Strept, pneumoniae, Strept. pyogenes); gram-negative Kokken (Neisseria gonorrhoeae) sowie gram-negative Stäbchen wie Enterobakteriaceen, z.B. Escherichia coli, Hämophilus influenzae, Citrobacter (Citrob. freundii, Citrob. divernis), Salmonella und Shigella; ferner Klebsiellen (Klebs. pneumoniae, Klebs. oxytoca), Enterobacter (Ent. aerogense, Ent. agglomerans), Hafnia, Serratia (Serr. marcescens), Proteus (Pr. mirabilis, Pr. rettgeri, Pr. vulgaris), Providencia, Yersinia, sowie die Gattung Acinetobacter. Darüber hinaus umfäßt das antibakterielle Spektrum die Gattung Pseudomonas (Ps. aeruginosa, Ps. maltophilia) sowie strikt anaerobe Bakterien wie z.B. Bacteroides fragilis, Vertreter der Gattung Peptococcus, Pentostreptococcus sowie die Gattung Clostridium; ferner Mykoplasmen (M. pneumoniae, M. hominis, M. urealyticum) sowie Mykobaktęrien, z.B. Mycobacterium tuberculosis.

Die obige Aufzählung von Erregern ist lediglich beispielhaft und keineswegs beschränkend aufzufassen. Als Krankheiten, die durch die genannten Erreger oder Mischinfektionen verursacht und durch die erfindungsgemäßen Verbindungen verhindert, gebessert oder geheilt werden können, seien beispielsweise genannt:
Infektionskrankheiten beim Menschen wie zum Beispiel Otitis, Pharyngitis, Pneumonie, Peritonitis, Pyelonephritis, Cystitis, Endocarditis, Systeminfektionen, Bronchitis (akut, chronisch), septische Infektionen, Erkrankungen der oberen Luftwege, diffuse Panbronchiolitis, pulmonäres Emphysem, Dysenterie, Enteritis, Leberabszesse, Urethritis, Prostatitis, Epididymitis, gastrointestinale Infektionen, Knochen- und Gelenkinfektionen, zystische Fibrose, Hautinfektionen, postoperative Wundinfektionen, Abszesse, Phlegmone, Wundinfektionen, infizierte Verbrennungen, Brandwunden, Infektionen im Mundbereich, Infektionen nach Zahnoperationen, Osteomyelitis, septische Arthritis, Cholecystitis, Peritonitis mit Appendicitis, Cholangitis, intraabdominale Abszesse, Pankreatitis, Sinusitis, Mastoiditis, Mastitis, Tonsillitis, Typhus, Meningitis und Infektionen des Nervensystems, Salpingitis, Endometritis, Genital-Infektionen, Pelveoperitonitis und Augeninfektionen.

Außer beim Menschen können bakterielle Infektionen auch bei anderen Spezies behandelt werden. Beispielhaft seien genannt:
Schwein: Coli-diarrhoe, Enterotoxämie, Sepsis, Dysenterie, Salmonellose, Metritis-Mastitis-Agalaktiae-Syndrom, Mastitis;
Wiederkäuer (Rind, Schaf, Ziege): Diarrhoe, Sepsis, Bronchopneumonie, Salmonellose, Pasteurellose, Mykoplasmose, Genitalinfektionen;
Pferd: Bronchopneumonien, Fohlenlähme, puerperale und postpuerperale Infektionen, Salmonellose;
Hund und Katze: Bronchopneumonie, Diarrhoe, Dermatitis, Otitis, Harnwegsinfekte, Prostatitis;
Geflügel (Huhn, Pute, Wachtel, Taube, Ziervögel und andere): Mycoplasmose, E. coli-Infektionen, chronische Luftwegserkrankungen, Salmonellose, Pasteurellose, Psittakose.

Ebenso können bakterielle Erkrankungen bei der Aufzucht und Haltung von Nutz- und Zierfischen behandelt werden, wobei sich das antibakterielle Spektrum über die vorher genannten Erreger hinaus auf weitere Erreger wie zum Beispiel Pasteurella, Brucella, Campylobacter, Listeria, Erysipelothrix, Corynebakterien, Borellia, Treponema, Nocardia, Rikettsien, Yersinia, erweitert.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitung in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seiein Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resportionsbeschleuniger, z.B.

quarternäre Ammoniumverbindungen, (g) Netzmittel, z.B. Cetylalkohol, Glycerinmonostearat, (h) Adsorptionsmittel, z.B. Kaolin und Bentonit und (i) Gleitmittel z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmitteln enthaltenden, Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxide, Calciumsilikat und Polyamidpulver oder Gemische dieser Stoffe. Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z.B. Waser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbesserte Zusätze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die genannten Zubereitungen können bei Mensch und Tier entweder oral, rektal, parenteral (intravenös, intramuskulär, subkutan), intracisternal, intravaginal, intraperitoneal, lokal (Puder, Salbe, Tropfen) und zur Therapie von Infektionen in Hohlräumen, Körperhöhlen angewendet werden. Als geeigete Zubereitungen kommen Injectionlösungen, Lösungen und Suspensionen für die orale Therapie, Gele, Aufgußformulierungen, Emulsionen, Salben oder Tropfen in Frage. Zur lokalen Therapie können ophtalmologische und dermatologische Formulierungen, Silber- und andere Salze, Ohrentropfen, Augensalben, Puder oder Lösungen verwendet werden. Bei Tieren kann die Aufnahme auch über das Futter oder Trinkwasser in geeigneten Formulierungen erfolgen. Ferner können Gele, Pulver, Puder, Tabletten, Retard-Tabletten, Premize, Konzentrate, Granulate, Pellets, Tabletten, Boli, Kapseln, Aerosole, Sprays, Inhalate bei Mensch und Tier angewendet werden. Ferner können die erfindungsgemäßen Verbindungen in andere Trägermaterialien wie zum Beispiel Kunststoffe, (Kunststoffketten zur lokalen Therapie), Kollagen oder Knochenzement eingearbeitet werden.

Im allgemeinen hat es sich sowhol in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff

auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die neuen Verbindungen können in den üblichen Konzentrationen und Zubereitungen zusammen mit dem Futter bzw. mit Futterzubereitungen oder mit dem Trinkwasser gegeben werden. Dadurch kann eine Infektion durch gram-negative oder gram-positive Bakterien verhindert, gebessert und/oder geheilt werden und dadurch eine Förderung des Wachstums und eine Verbesserung der Verwertung des Futters erreicht werden.

Die Temperaturangaben in den nachfolgenden Beispielen sind in °C.

Herstellung der Ausgangsverbindungen

Beispiel A

N-(2-Nitrophenylsulfenyl)-bis(2-hydroxyethyl)-amin (2)

Eine Lösung von 210 g (2 mol) Bis(2-hydroxyethyl)-amin und 202 g (2 mol) Triethylamin in 3 l Dichlormethan wird innerhalb 90 Minuten mit einer Lösung von 379 g (2 mol) 2-Nitrophenylsulfenylchlorid in 2 l Dichlormethan versetzt, wobei die Temperatur von 21° auf 34° ansteigt. Man läßt 2 Stunden bei Raumtemperatur nachrühren, läßt über Nacht stehen und wäscht die Lösung sechsmal mit je 1 l Wasser, trocknet mit Natriumsulfat und engt im Vakuum ein. Der Rückstand ist ein orange-gelbes Festprodukt vom Schmelzpunkt 82-86°C, das für die weitere Umsetzung ausreichend rein ist. Ausbeute: 499 g.

Beispiel B

N-(2-Nitrophenylsulfenyl)-bis[2-(4-toluolsulfonyloxy)-ethyl]-amin (3)

389 g (1,5 mol) rohes N-(2-Nitrophenylsulfenyl)-bis(2-hydroxyethyl)-amin werden in 2 l absoluten Pyridins gelöst und bei -10° mit 580 g (3 mol) 4-Toluolsulfonsäurechlorid innerhalb 20 Minuten versetzt. Man rührt 4 Stunden bei -5° bis 0°, läßt über Nacht bei 0° stehen und versetzt mit 4 l Wasser. Danach wird mit 3 x 1 l Dichlormethan extrahiert und der Extrakt mit 7 x 1 l Wasser gewaschen, mit Natriumsulfat getrocknet und eingeengt. Der ölige Rückstand wird mit 1 l einer Mischung aus Acetonitril/Methanol (1:1) verrührt, wobei ein Festprodukt ausfällt, das abgesaugt und mit Methanol gewaschen wird.

Ausbeute: 423,6 g vom Schmelzpunkt 91-93°.

Beispiel C

1-Cyclopropyl-4-(2-nitrophenylsulfenyl)-piperazin

Eine Mischung von 112 g (0,198 mol) N-(2-Nitrophenylsulfenyl)-bis[2-(4-toluolsulfonyloxy)-ethyl]-amin, 16,7 g (0,29 mol) Cyclopropylamin und 82 g (0,6 mol) pulverisiertem Kaliumcarbonat in 3,3 l Isopropanol wird 6 Stunden unter Rückfluß erhitzt. Danach wird im Vakuum eingeengt, der Rückstand in 550 ml Dichlormethan aufgenommen, mit Wasser gewaschen, mit Natriumsulfat getrocknet und eingeengt. Zur Reinigung wird das Rohprodukt mit Dichlormethan als Laufmittel über 600 g Kieselgel filtriert und das Eluat eingeengt.

Ausbeute: 47 g vom Schmelzpunkt 100-102°.

Analog werden folgende 1-substituierte 4-(2-Nitrophenylsulfenyl)-piperazine (4) erhalten:

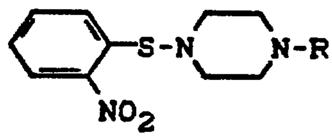 (4)

| R | Schmelzpunkt |
|---|---|
| $(CH_3)_3C$ | 103-105° |
| $(HO-CH_2)_3C$ | 179-182° |
| 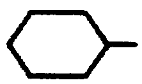 | 182-186° |
| 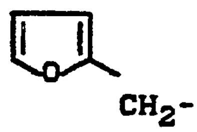 | oranges Öl |
| 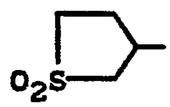 | oranges Öl |

Beispiel D

1-Cyclopropyl-piperazin

Eine Lösung von 19 g (68 mmol) 1-Cyclopropyl-4-(2-nitrophenyl)-piperazin in 135 ml Dichlormethan wird mit einer Lösung von 11,3 g (68 mmol) 2-Mercaptobenthiazol in 80 ml Dichlormethan/40 ml Methanol versetzt und dann mit 136 ml 1n-Salzsäure angesäuert. Die Mischung wird mit 3 mal 50 ml Wasser extrahiert, die vereinigten wäßrigen Phasen eingeengt und der Rückstand mit etwas Ethanol aufgeschlämmt, abgesaugt und getrocknet.

Ausbeute: 12,8 g (96,5 % der Theorie) 1-Cyclopropyl-piperazin-dihydrochlorid vom Schmelzpunkt etwa 250° (unter Zersetzung) (verfärbt sich bereits ab etwa 210°).

Zur Herstellung des freien 1-Cyclopropyl-piperazins werden 11,9 g des Dihydrochlorids mit einer Lösung von 8,4 g Kaliumhydroxid in 60 ml Methanol 3 Stunden bei Raumtemperatur verrührt und von ungelöstem Kaliumchlorid abgesaugt. Aus der Mutterlauge wird das Methanol bei Normaldruck abdestilliert und der Rückstand im Vakuum destilliert.

Ausbeute: 7,0 g (92,6 % der Theorie) 1-Cyclopropyl-piperazin vom Siedepunkt 50°/12 mbar.

Analog werden folgende 1-substituierte Piperazin-Dihydrochloride (5) beziehungsweise 1-substituierten Piperazine (III) erhalten:

| R | 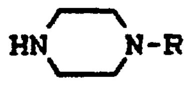 x 2 HCl<br>(5)<br>Schmelzpunkt | 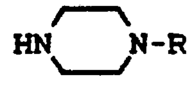<br>(III)<br>Siedepunkt |
|---|---|---|
| $(CH_3)_3C$ | >300° (Zersetzung) | |
| $(HO-CH_2)_3C$ | 222-226° (Zersetzung) | |
| 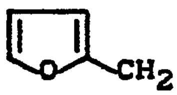 | 245-250° (Zersetzung) | 82° /0,3mbar |
| 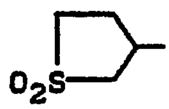 | 240-245° (Zersetzung) | |

Beispiel E

1-(6-Hydroxy-2,2-dimethyl-1,3-dioxepan-5-yl)-piperazin

14,4 g (0,1 mol) 4,4-Dimethyl-3,5,8-trioxabicyclo[5,1,0] octan (J. Org. Chem. 41,2469 [1976]) werden in 100 ml Acetonitril mit 20 g (0,1 mol) Piperazinhexahydrat 8 Stunden unter Rückfluß erhitzt und vom ausgefallenen 1,4-Bis(6-Hydroxy-2,2-dimethyl-1,3-dioxepan-5-yl)-piperazin [Schmelzpunkt: 235-237° (aus Isopropanol)] abgesaugt. Die Mutterlauge wird eingeengt und der Rückstand mit Dichlormethan/Methanol (9:1) als Laufmittel auf 200 g Kieselgel chromatographisch gereinigt, wobei die Hauptkomponente gegen Ende der Chromatographie mit Dichlormethan/Methanol (4:1) eluiert wird. Man erhält 4,7 g 1-(6-Hydroxy-2,2-dimethyl-1,3-dioxepan-5-yl)-piperazin als Öl; $R_f$-Wert = 0,05 [auf Kieselgel; Dichlormethan/Methanol (9:1)].

Beispiel F

1-(6-Hydroxy-3-cyclohexen-1-yl)-piperazin

Analog Beispiel E erhält man mit 1,4-Cyclohexadienmonoepoxid neben 1,4-Bis(6-hydroxy-3-cylcohexen-1-yl)-piperazin (Schmelzpunkt = 207-209°) 1-(6-Hydroxy-3-cyclohexen-1-yl)-piperazin vom Schmelzpunkt 58-64°.

Beispiel 1

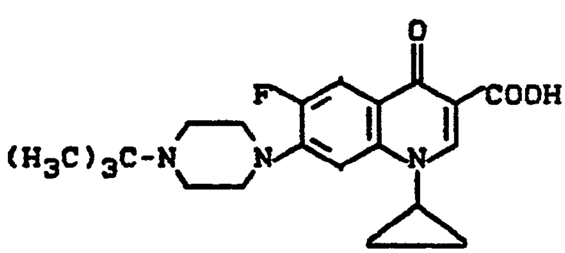

Eine Mischung aus 1,33 g (5 mmol) 1-Cyclopropyl-6,7-difluor-1,4-dihydro-4-oxo-3-chinolincarbonsäure und 1,1 g (5 mmol) 1-(tert.-Butyl)-piperazin-Dihydrochlorid in 12,5 ml Pyridin wird mit 2,2 g (20 mmol) 1,4-Diazabicyclo [2,2,2]octan versetzt und 3 Stunden unter Rückfluß erhitzt. Man engt im Vakuum ein, verrührt den Rückstand mit 25 ml Wasser und stellt mit 2n-Salzsäure auf pH5. Der ausgefallene Niederschlag wird abgesaugt, mit Wasser gewaschen und aus Glykolmonomethylether umkristallisiert. Man erhält 1,45 g (75 % der Theorie) 7-(4-tert.-Butyl-1-piperazinyl)-1-cyclopropyl-6-fluor-1,4-dihydro-4-oxo-3-chinolincarbonsäure vom Schmelzpunkt 257-260°.

Beispiel 2

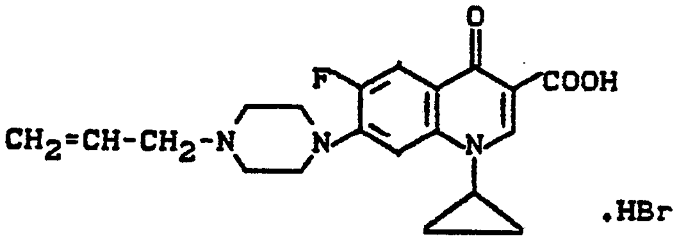

3,3 g (10 mmol) 1-Cyclopropyl-6-fluor-1,4-dihydro-4-oxo-7-(1-piperazinyl)-3-chinolincarbonsäure werden in 50 ml Dimethylformamid mit 1,8 g (15 mmol) Allylbromid und 2,1 g Triethylamin 8 Stunden auf 60° erhitzt. Die Mischung wird im Vakuum eingeengt, der Rückstand mit 30 ml Wasser verrührt, der Niederschlag abgesaugt, mit Methanol gewaschen und getrocknet. Man erhält 2,2 g 7-(4-Allyl-1-piperazinyl)-1-cyclopropyl-6-fluor-1,4-dihydro-4-oxo-3-chinolincarbonsäure-Hydrobromid vom Schmelzpunkt 295-298° (unter Zersetzung); nach Umkristallisation aus Wasser isoliert man 1,5 g vom Schmelzpunkt 300-302° (unter Zersetzung).

Beispiel 3

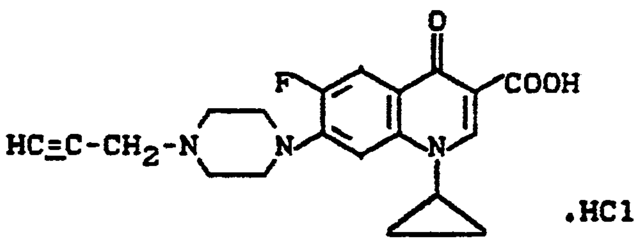

Analog Beispiel 2 erhält man mit Proparglychlorid 1-Cyclopropyl-6-fluor-1,4-dihydro-4-oxo-7-(4-propargyl-1-piperazinyl)-3-chinolincarbonsäure-Hydrochlorid vom Schmelzpunkt 237-240° (unter Zersetzung).

Beispiel 4

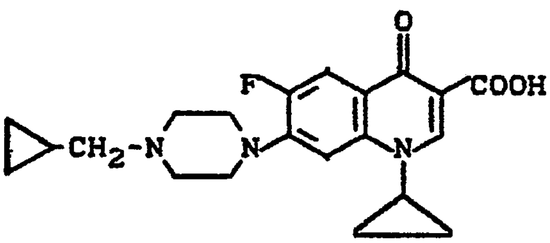

3,3 g (10 mmol) 1-Cyclopropyl-6-fluor-1,4-dihydro-4-oxo-7-(1-piperazinyl)-3-chinolincarbonsäure werden in 50 ml Dimethylformamid mit 1,8 g (20 mmol) Chormethylcyclopropan, 3,3 g (20 mmol) Kaliumjodid und 2,1 g Triethylamin 5 Stunden auf 90°erhitzt. Die Lösung wird in 40 ml Wasser eingegossen, mit 2n-Salzsäure auf pH5 gestellt, der Niederschlag abgesaugt und aus Methanol umkristallisiert. Man erhält 0,6 g 1-Cyclopropyl-7-(4-cyclopropylmethyl-1-piperazinyl)-6-fluor-1,4-dihydro-4-oxo-3-chinolincarbonsäure vom Schmelzpunkt 279-282° (unter Zersetzung).

Beispiel 5

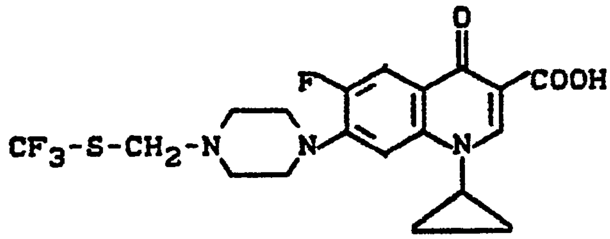

3,3 g (10 mmol) 1-Cyclopropyl-6-fluor-1,4-dihydro-4-oxo-7-(1-piperazinyl)-3-chinolincarbonsäure werden in 50 ml Dimethylformamid mit 4 g (27 mmol) Trifluormethylthiomethychlorid, 2,1 g Triethylamin, 1,7 g Kaliumjodid und 0,2 g Tetrabutylammoniumjodid 16 Stunden auf 90° erhitzt. Die Lösung wird in 50 ml Wasser gegossen, der Niederschlag abgesaugt und mit Methanol aufgekocht. Man erhält 1,5 g 1-Cyclopropyl-6-fluor-1,4-dihydro-4-oxo-7-(4-trifluormethylthiomethyl-1-piperazinyl)-3-chinolincarbonsäure vom Schmelzpunkt 247-250° (unter Zersetzung).

Beispiel 6

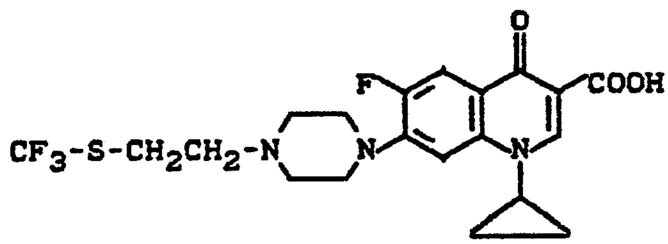

Analog Beispiel 5 setzt man 2-Trifluormethylthio-ethylbromid um und erhält 1-Cyclopropyl-6-fluor-1,4-dihydro-4-oxo-7-[4-(2-trifluormethylthioethyl)-1-piperazinyl]-3-chinolincarbonsäure vom Schmelzpunkt 188-190° (unter Zersetzung).

Beispiel 7

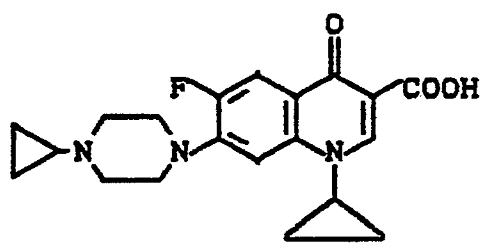

2,8 g (10 mmol) 7-Chlor-1-cyclopropyl-6-fluor-1,4-dihydro-4-oxo-3-chinolincarbonsäure werden in 25 ml Dimethylsulfoxid mit 1,3 g 1-Cyclopropylpiperazin und 2,2 g (20 mmol) 1,4-Diazabicyclo[2,2,2]-octan versetzt und 5 Stunden auf 140° erhitzt. Die Suspension wird mit 40 ml Wasser versetzt, der Niederschlag abgesaugt, mit Wasser gewaschen und mit 20 ml Methanol aufgekocht. Man erhält 1,2 g 1-Cyclopropyl-7-(4-cyclopropyl-1-piperazinyl)-6-fluor-1,4-dihydro-4-oxo-3-chinolincarbonsäure vom Schmelzpunkt 226-229° (unter Zersetzung).

Beispiel 8

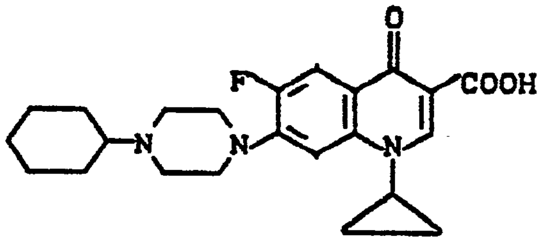

Analog Beispiel 7 erhält man mit 1-Cyclohexyl-piperazin 7-(4-Cyclohexyl-1-piperazinyl)-1-cyclopropyl-6-fluor-1,4-dihydro-4-oxo-3-chinolincarbonsäure vom Schmelzpunkt 232-236°.

Beispiel 9

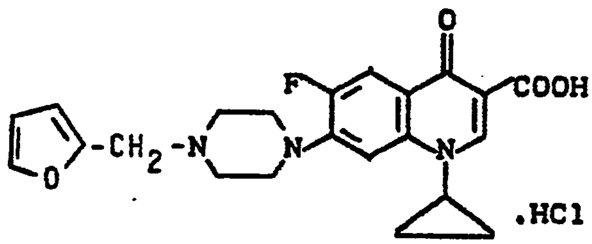

.HCl

Analog Beispiel 7 erhält man mit 1-(2-Furylmethyl)-piperazin 1-Cyclopropyl-6-fluor-7-[4-(2-furylmethyl)-1-piperazinyl]-1,4-dihydro-4-oxo-3-chinolincarbonsäure vom Schmelzpunkt 210-215° (unter Zersetzung), die durch Lösen in verdünnter Salzsäure (1:1) und Ausfällen mit Methanol in 1-Cyclopropyl-6-fluor-7-[4-(2-furylmethyl)-1-piperazinyl]-1,4-dihydro-4-oxo-3-chinolincarbonsäure-Hydrochlorid vom Schmelzpunkt 294-297° (unter Zersetzung) übergeführt wird.

Beispiel 10

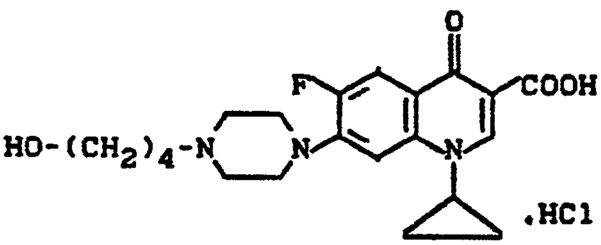

.HCl

Analog Beispiel 5 setzt man 4-Chlor-1-butanol um, behandelt das Reaktionsprodukt mit verdünnter Salzsäure (1:1) und erhält 1-Cyclopropyl-6-fluor-1,4-dihydro-7-[4-(4-hydroxybutyl)-1-piperazinyl]-4-oxo-3-chinolincarbonsäure-Hydrochlorid vom Schmelzpunkt 325° (unter Zersetzung).

Beispiel 11

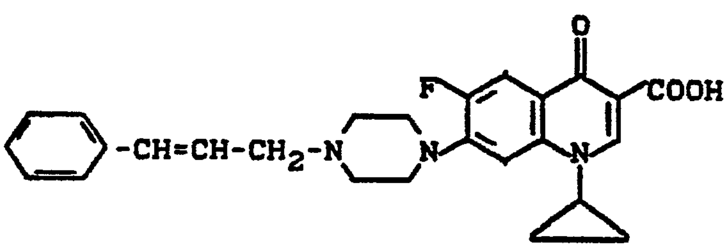

2,8 g (10 mmol) 7-Chlor-1-cyclopropyl-6-fluor-1,4-dihydro-4-oxo-3-chinolincarbonsäure werden in 35 ml Dimethylsulfoxid mit 2,1 g (10 mmol) 1-Cinnamyl-piperazin und 2,2 g 1,4-Diazabicyclo[2,2,2]octan versetzt und 5 Stunden auf 140° erhitzt. Die Mischung wird im Hochvakuum eingeengt, mit 50 ml Wasser

verrührt und mit 2n-Salzsäure auf pH6 eingestellt. Der ausgefallene Niederschlag wird abgesaugt, mit Wasser gewaschen und mit Methanol aufgekocht. Man erhält 1,7 g 7-(4-Cinnamyl-1-piperazinyl)-1-cyclopropyl-6-fluor-1,4-dihydro-4-oxo-3-chinolincarbonsäure vom Schmelzpunkt 230-234° (unter Zersetzung).

Beispiel 12

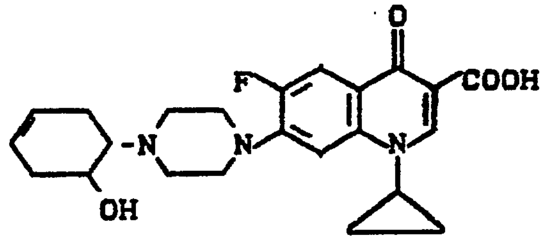

Analog Beispiel 11 erhält man mit 1-(6-Hydroxy-3-cyclohexen-1-yl)-piperazin 1-Cyclopropyl-6-fluor-1,4-dihydro-7-[4-(6-hydroxy-3-cyclohexen-1-yl)-1-piperazinyl]-4-oxo-3-chinolincarbonsäure vom Schmelzpunkt 277-280°.

Beispiel 13

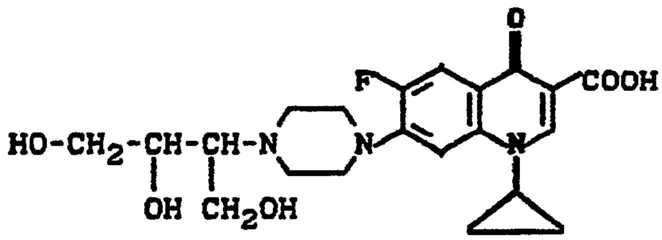

2,8 g (10 mmol) 7-Chlor-1-cyclopropyl-6-fluor-1,4-dihydro-4-oxo-3-chinolincarbonsäure werden in 30 ml Dimethysulfoxid mit 2,5 g (11 mmol) 1-(6-Hydroxy-2,2-dimethyl-1,3-dioxepan-5-yl)-piperazin und 2,25 g (20 mmol) 1,4-Diazabicyclo[2,2,2]octan 4 Stunden auf 140° erhitzt, das Reaktionsgemisch wird eingeengt, das erhaltene Öl mit 20 ml Methanol verdünnt und mit 2n-Salzsäure auf pH 5 eingestellt. Der ausgefallene Niederschlag wird aus Glykolmonomethylether/Methanol unkristallisiert. Man erhält 0,5 g 1-Cyclopropyl-6-fluor-1,4-dihydro-7-[4-(6-hydroxy-2,2-dimethyl-1,3-dioxepan-5-yl)-1-piperazinyl]-4-oxo-3-chinolincarbonsäure vom Schmelzpunkt 255-262° (unter Zersetzung), die zur Abspaltung der Schutzgruppe in 10 ml 2n Salzsäure unter Erwärmen gelöst werden. Nach 1-stündigem Stehen bei Raumtemperatur waren 0,3 g 1-Cyclopropyl-6-fluor-1,4-dihydro-7-[4-(1,3,4-trihydroxy-2-butyl)-1-piperazinyl]-4-oxo-3-chinolincarbonsäure vom Schmelzpunkt 262-268° (unter Zersetzung) ausgefallen.

Beispiel 14

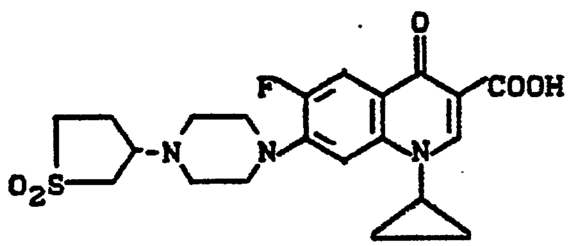

Analog Beispiel 11 erhält man mit 1-(1,1-Dioxido-tetrahydrothiophen-3-yl)-piperazin 1-Cyclopropyl-7-[4-(1,1-dioxido-tetrahydrothiophen-3-yl)-1-piperazinyl]-6-fluor-1,4-dihydro-4-oxo-3-chinolincarbonsäure vom Schmelzpunkt 252-255° (unter Zersetzung).

**Patentansprüche**

1. 1-Cyclopropyl-6-fluor-1,4-dihydro-4-oxo-7-(1-piperazinyl)-3-chinolincarbonsäuren der Formel (I)

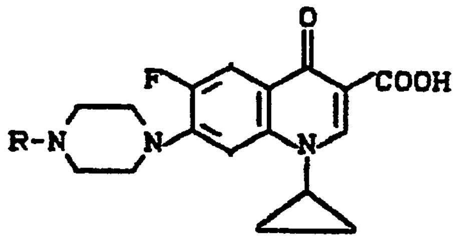

(I)

in welcher
R für verzweigtes oder unverzweigtes durch Hydroxy oder Methoxy zwei- bis dreifach substituiertes Propyl, verzweigtes oder unverzweigtes, durch Hydroxy oder Methoxy 1-bis 3-fach substituiertes Butyl und für unsubstituiertes tert.-Butyl steht, ferner für 2-Methylthioethyl, Trifluormethylthiomethyl, 2-Trifluormethylthioethyl, Cycloalkyl mit 3 bis 6 Kohlenstoffatomen, gegebenenfalls durch Hydroxy substituiertes Cycloalkenyl mit 5 oder 6 Kohlenstoffatomen, 1,1-Dioxidotetrahydrothiophen-3-yl, Cyclopropylmethyl, 1-Phenylethyl, Furylmethyl sowie für gegebenenfalls durch Phenyl substituiertes Allyl und Propargyl steht,
und deren pharmazeutisch verwendbaren Hydrate, Säureadditionssalze, Alkali-, Erdalkali-, Silber- und Guanidiniumsalze sowie ihre Ester und andere übliche Prodrug-Formen.

2. 1-Cyclopropyl-6-fluor-1,4-dihydro-4-oxo-7-(1-piperazinyl)-3-chinolincarbonsäuren der Formel (I)

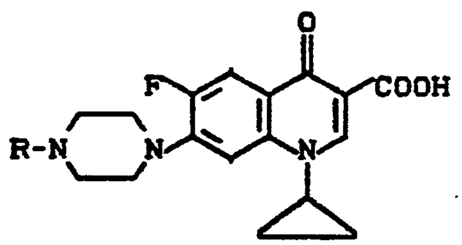

(I)

in welcher
R für verzweigtes oder unverzweigtes durch Hydroxy oder Methoxy zwei- bis dreifach substituiertes Propyl, verzweigtes oder unverzweigtes, durch Hydroxy oder Methoxy 1-bis 3-fach substituiertes Butyl und für unsubstituiertes tert.-Butyl steht, ferner für Trifluormethylthiomethyl, 2-Trifluormethylthioethyl, Cycloalkyl mit 3 bis 6 Kohlenstoffatomen, gegebenenfalls durch Hydroxy substituiertes Cyclohexenyl, 1,1-Dioxidotetrahydrothiophen-3-yl, Cyclopropylmethyl, Furylmethyl sowie für gegebenenfalls durch Phenyl substituiertes Allyl und Propargyl steht,
und deren pharmazeutisch verwendbaren Hydrate, Säureadditionssalze, Alkali-, Erdalkali-, Silber- und Guanidiniumsalze sowie ihre Ester und andere übliche Prodrug-Formen.

3. Verfahren zur Herstellung von 1-Cyclopropyl-6-fluor-1,4-dihydro-4-oxo-7-(1-piperazinyl)-3-chinolincarbonsäuren der Formel (I)

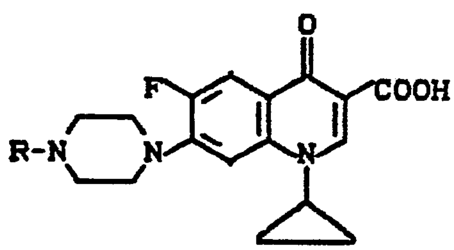

(I)

in welcher
R für verzweigtes oder unverzweigtes durch Hydroxy oder Methoxy zwei- bis dreifach substituiertes Propyl, verzweigtes oder unverzweigtes, durch Hydroxy oder Methoxy 1-bis 3-fach substituiertes Butyl und für unsubstituiertes tert.-Butyl steht, ferner für 2-Methylthioethyl, Trifluormethylthiomethyl, 2-Trifluormethylthioethyl, Cycloalkyl mit 3 bis 6 Kohlenstoffatomen, gegebenenfalls durch Hydroxy substituiertes Cycloalkenyl mit 5 oder 6 Kohlenstoffatomen, 1,1-Dioxidotetrahydrothiophen-3-yl, Cyclopropylmethyl, 1-Phenylethyl, Furylmethyl sowie für gegebenenfalls durch Phenyl substituiertes Allyl und Propargyl steht,
dadurch gekennzeichnet, daß man Verbindungen der Formel (II)

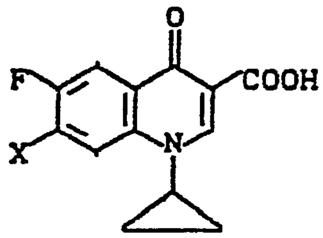 (II),

in welcher
X für Fluor oder Chlor steht,
mit Piperazinderivaten der Formel (III)

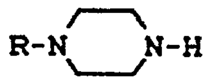 (III)

in welcher
R die oben angegebene Bedeutung hat, gegebenenfalls in Gegenwart von Säurebindern umsetzt.

4. Verfahren nach Anspruch 3, dadurch gekennzeichnet, daß man auf 1 Mol der Chinoloncarbonsäure (II) 1 bis 15 Mol des Piperazins (III) einsetzt.

5. Verfahren zur Herstellung von 1-Cyclopropyl-6-fluor-1,4-dihydro-4-oxo-7-(1-piperazinyl)-3-chinolincarbonsäure der Formel (I)

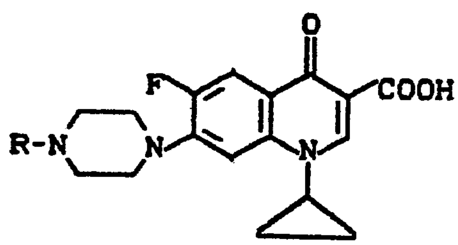 (I)

in welcher
R für verzweigtes oder unverzweigtes durch Hydroxy oder Methoxy zwei- bis dreifach substituiertes Propyl, verzweigtes oder unverzweigtes, durch Hydroxy oder Methoxy 1-bis 3-fach substituiertes Butyl und für unsubstituiertes tert.-Butyl steht, ferner für 2-Methylthioethyl, Trifluormethylthiomethyl, 2-Trifluormethylthioethyl, Cycloalkyl mit 3 bis 6 Kohlenstoffatomen, gegebenenfalls durch Hydroxy substituiertes Cycloalkenyl mit 5 oder 6 Kohlenstoffatomen, 1,1-Dioxidotetrahydrothiophen-3-yl, Cyclopropylmethyl, 1-Phenylethyl, Furylmethyl sowie für gegebenenfalls durch Phenyl substituiertes Allyl und Propargyl steht,
dadurch gekennzeichnet, daß man 1-Cyclopropyl-6-fluor-1,4-dihydro-4-oxo-7-(1-piperazinyl)-3-chinolincarbonsäure der Formel (IV)

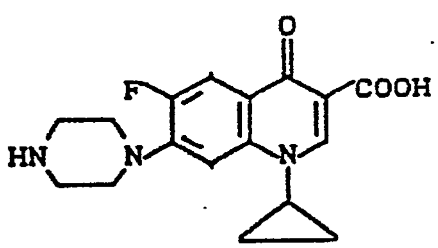 (IV)

mit Verbindungen der Formel (V)

R–Y (V),

in welcher
R die oben angegebene Bedeutung hat, jedoch nicht tert.-Butyl-Tris(hydroxymethyl)-methyl oder Cycloalkyl sein kann, und
Y Halogen, insbesondere Chlor, Brom oder Jod bedeutet,
gegebenenfalls in Gegenwart von Säurebindern umsetzt.

6. Verfahren nach Anspruch 5, dadurch gekennnzeichnet, daß man auf 1 Mol der Verbindung (IV) 1 bis 4 Mol der Verbindung (V) einsetzt.

7. Verfahren nach Anspruch 5, dadurch gekennzeichnet, daß es in Gegenwart von Phasentransferkatalysatoren in einem Verdünnungsmittel durchgeführt wird.

8. 1-Cyclopropyl-6-fluor-1,4-dihydro-4-oxo-7-(1-piperazinyl)-3-chinolincarbonsäuren der Formel (I)

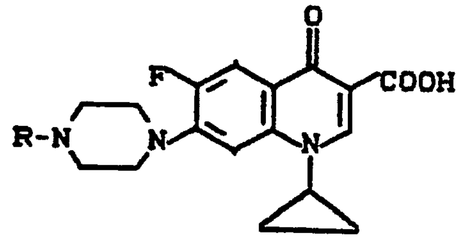

(I)

in welcher
R für verzweigtes oder unverzweigtes durch Hydroxy oder Methoxy zwei- bis dreifach substituiertes Propyl, verzweigtes oder unverzweigtes, durch Hydroxy oder Methoxy 1-bis 3-fach substituiertes Butyl und für unsubstituiertes tert.-Butyl steht, ferner für 2-Methylthioethyl, Trifluormethylthiomethyl, 2-Trifluormethylthioethyl, Cycloalkyl mit 3 bis 6 Kohlenstoffatomen, gegebenenfalls durch Hydroxy substituiertes Cycloalkenyl mit 5 oder 6 Kohlenstoffatomen, 1,1-Dioxidotetrahydrothiophen-3-yl, Cyclopropylmethyl, 1-Phenylethyl, Furylmethyl sowie für gegebenenfalls durch Phenyl substituiertes Allyl und Propargyl steht,
und deren pharmazeutisch verwendbaren Hydrate, Säureadditionssalze, Alkali-, Erdalkali-, Silber- und Guanidiniumsalze sowie ihre Ester und andere übliche Prodrug-Formen, zur Verwendung in einem Verfahren zur therapeutischen Behandlung des menschlichen oder tierischen Körpers.

9. Arzneimittel, enthaltend 1-Cyclopropyl-6-fluor-1,4-dihydro-4-oxo-7-(1-piperazinyl)-3-chinolincarbonsäuren der Formel (I)

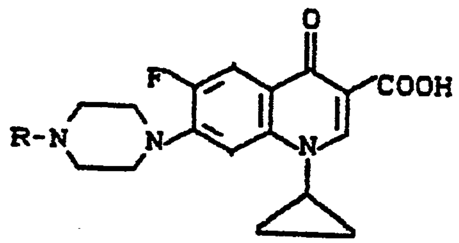

(I)

in welcher
R für verzweigtes oder unverzweigtes durch Hydroxy oder Methoxy zwei- bis dreifach substituiertes Propyl, verzweigtes oder unverzweigtes, durch Hydroxy oder Methoxy 1-bis 3- fach substituiertes Butyl und für unsubstituiertes tert.-Butyl steht, ferner für 2-Methylthioethyl, Trifluormethylthiomethyl, 2-Trifluormethylthioethyl, Cycloalkyl mit 3 bis 6 Kohlenstoffatomen, gegebenenfalls durch Hydroxy substituiertes Cycloalkenyl mit 5 oder 6 Kohlenstoffatomen, 1,1-Dioxidotetrahydrothiophen-3-yl, Cyclopropylmethyl, 1-Phenylethyl, Furylmethyl sowie für gegebenenfalls durch Phenyl substituiertes Allyl und Propargyl steht,
und deren pharmazeutisch verwendbaren Hydrate, Säureadditionssalze, Alkali-, Erdalkali-, Silber- und Guanidiniumsalze sowie ihre Ester und andere übliche Prodrug-Formen.

10. Verwendung von 1-Cyclopropyl-6-fluor-1,4-dihydro-4-oxo-7-(1-piperazinyl)-3-chinolincarbonsäuren der Formel (I)

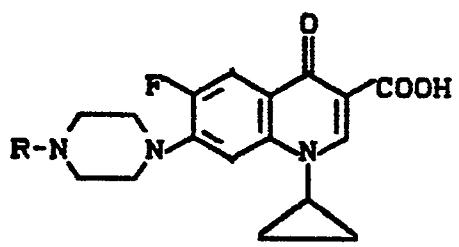

(I)

in welcher
R für verzweigtes oder unverzweigtes durch Hydroxy oder Methoxy zwei- bis dreifach substituiertes Propyl, verzweigtes oder unverzweigtes, durch Hydroxy oder Methoxy 1-bis 3-fach substituiertes Butyl und für unsubstituiertes tert.-Butyl steht, ferner für 2-Methylthioethyl, Trifluormethylthiomethyl, 2-Trifluoromethylthioethyl, Cycloalkyl mit 3 bis 6 Kohlenstoffatomen, gegebenenfalls durch Hydroxy substituiertes Cycloalkylenyl mit 5 oder 6 Kohlenstoffatomen, 1,1-Dioxidotetrahydrothiophen-3-yl, Cyclopropylmethyl, 1-Phenylethyl, Furylmethyl sowie für gegebenenfalls durch Phenyl substituiertes Allyl und Propargyl steht,

und deren pharmazeutisch verwendbaren Hydraten, Säureadditionssalzen, Alkali-, Erdalkali-, Silber- und Guanidiniumsalzen sowie ihren Estern und anderen üblichen Prodrug-Formen zur Herstellung von Arzneimitteln.

**Claims**

1. 1-Cyclopropyl-6-fluoro-1,4-dihydro-4-oxo-7-(1-piperazinyl)-3-quinolinecarboxylic acids of the formula (I)

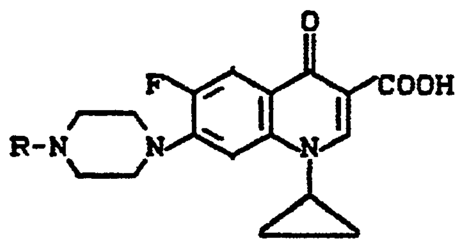

(I)

in which
R represents branched or straight-chain propyl which is di- or trisubstituted by hydroxyl or methoxy, or branched or straight-chain butyl which is mono-, di- or trisubstituted by hydroxyl or methoxy, or represents unsubstituted tert.-butyl, or furthermore represents 2-methylthioethyl, trifluoromethylthiomethyl, 2-trifluoromethylthioethyl, cycloalkyl with 3 to 6 carbon atoms, cycloalkenyl which has 5 or 6 carbon atoms and is optionally substituted by hydroxyl, 1,1-dioxidotetrahydrothiophen-3-yl, cyclopropylmethyl, 1-phenethyl or furylmethyl, or represents allyl or propargyl which is optionally substituted by phenyl,
and pharmaceutically usable hydrates, acid addition salts and alkali metal, alkaline earth metal, silber and guanidinium salts thereof, and their esters and other customary prodrug forms.

2. 1-Cyclopropyl-6-fluoro-1,4-dihydro-4-oxo-7-(1-piperazinyl)-3-quinolinecarboxylic acids of the formula (I)

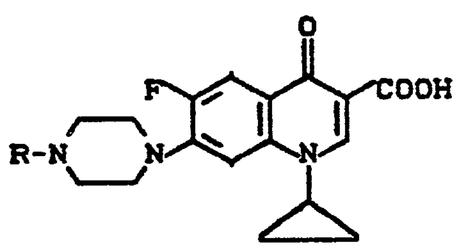

(I)

in which
R represents branched or straight-chain propyl which is di- or trisubstituted by hydroxyl or methoxy, or branched or straight-chain butyl which is mono-, di- or trisubstituted by hydroxyl or methoxy, or represents unsubstituted tert.-butyl, or furthermore represents trifluoromethylthiomethyl, 2-trifluoromethylthioethyl, cycloalkyl with 3 to 6 carbon atoms, cyclohexenyl which is optionally substituted by hydroxyl, 1,1-dioxidotetrahydrothiophen-3-yl, cyclopropylmethyl or furylmethyl, or represents allyl or propargyl which is optionally substituted by phenyl,
and pharmaceutically usable hydrates, acid addition salts and alkali metal, alkaline earth metal, silver and guanidinium salts thereof, and their esters and other customary prodrug forms.

3. Process for the preparation of 1-cyclopropyl-6-fluoro-1,4-dihydro-4-oxo-7-(1-piperazinyl)-3-quinolinecarboxylic acids of the formula (I)

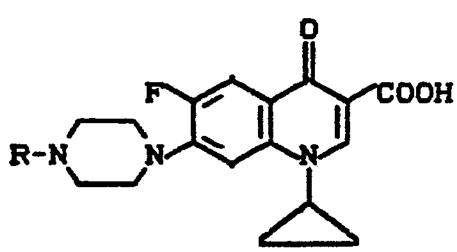

(I)

in which
R represents branched or straight-chain propyl which is di- or trisubstituted by hydroxyl or methoxy, or branched or straight-chain butyl which is mono-, di- or trisubstituted by hydroxyl or methoxy, or represents unsubstituted tert.-butyl, or furthermore represents 2-methylthioethyl, trifluoromethylthiomethyl,

2-trifluoromethylthioethyl, cycloalkyl with 3 to 6 carbon atoms, cycloalkenyl which has 5 or 6 carbon atoms and is optionally substituted by hydroxyl, 1,1-dioxidotetrahydrothiophen-3-yl, cyclopropylmethyl, 1-phenethyl or furylmethyl, or represents allyl or propargyl which is optionally substituted by phenyl, characterized in that compounds of the formula (II)

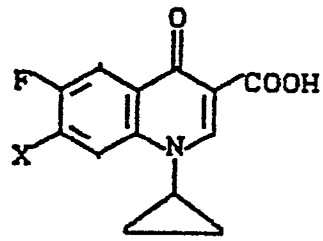

(II)

in which
X represents fluorine or chlorine are reacted with piperazine derivatives of the formula (III)

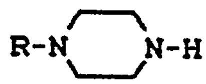

(III)

in which
R has the abovementioned meaning, if appropriate in the presence of acid-binding agents.

4. Process according to Claim 3, characterized in that 1 to 15 mol of the piperazine (III) are employed per mol of quinolonecarboxylic acid (II).

5. Process for the preparation of 1-cyclopropyl-6-fluoro-1,4-dihydro-4-oxo-7-(1-piperazinyl)-3-quinolinecarboxylic acids of the formula (I)

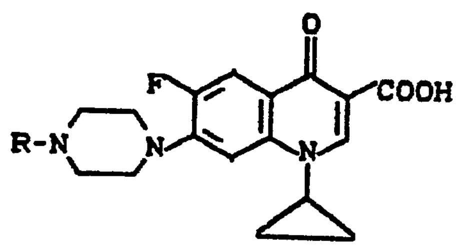

(I)

in which
R represents branched or straight-chain propyl which is di- or trisubstituted by hydroxyl or methoxy, or branched or straight-chain butyl which is mono-, di- or trisubstituted by hydroxyl or methoxy, or represents unsubstituted tert.-butyl, or furthermore represents 2-methylthioethyl, trifluoromethylthiomethyl, 2-trifluoromethylthioethyl, cycloalkyl with 3 to 6 carbon atoms, cycloalkenyl which has 5 or 6 carbon atoms and is optionally substituted by hydroxyl, 1,1-dioxidotetrahydrothiophen-3-yl, cyclopropylmethyl, 1-phenethyl or furylmethyl, or represents allyl or propargyl which is optionally substituted by phenyl, characterized in that 1-cyclopropyl-6-fluoro-1,4-dihydro-4-oxo-7-(1-piperazinyl)-3-quinolinecarboxylic acid of the formula (IV)

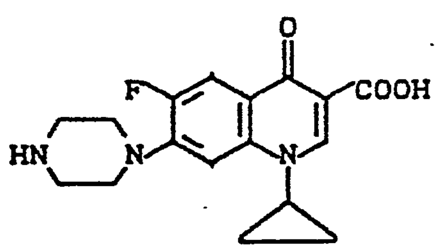

(IV)

is reacted with compounds of the formula (V)

R–Y (V)

in which
R has the abovementioned meaning but cannot be tert.-butyl, tris(hydroxymethyl)-methyl or cycloalkyl, and
Y denotes halogen, in particular chlorine, bromine or iodine,
if appropriate in the presence of acid-binding agents.

6. Process according to Claim 5, characterized in that 1 to 4 mol of the compound (V) are employed per mol of the compound (IV).

7. Process according to Claim 5, characterized in that it is carried out in the presence of phase transfer catalysts in a diluent.

8. 1-Cyclopropyl-6-fluoro-1,4-dihydro-4-oxo-7-(1-piperazinyl)-3-quinolinecarboxylic acids of the formula (I)

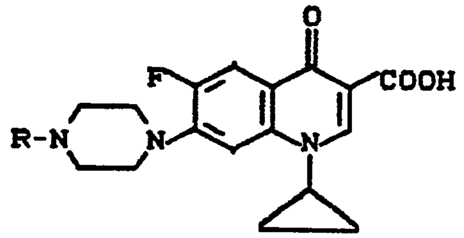

(I)

in which
R represents branched or straight-chain propyl which is di- or trisubstituted by hydroxyl or methoxy, or branched or straight-chain butyl which is mono-, di- or trisubstituted by hydroxyl or methoxy, or represents unsubstituted tert.-butyl, or furthermore represents 2-methylthioethyl, trifluoromethylthiomethyl, 2-trifluoromethylthioethyl, cycloalkyl with 3 to 6 carbon atoms, cycloalkenyl which has 5 or 6 carbon atoms and is optionally substituted by hydroxyl, 1,1-dioxidotetrahydrothiophen-3-yl, cyclopropylmethyl, 1-phenethyl or furylmethyl, or represents allyl or propargyl which is optionally substituted by phenyl, and pharmaceutically usable hydrates, acid addition salts and alkali metal, alkaline earth metal, silber and guanidinium salts thereof, and their esters and other customary prodrug forms, for use in a method for the therapeutic treatment of the human or animal body.

9. Medicaments containing 1-cyclopropyl-6-fluoro-1,4-dihydro-4-oxo-7-(1-piperazinyl)-3-quinolinecarboxylic acids of the formula (I)

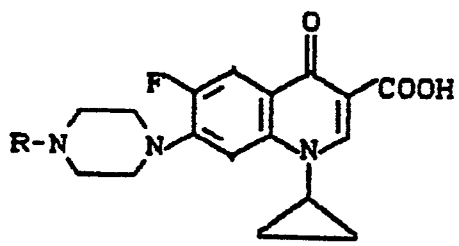

(I)

in which
R represents branched or straight-chain propyl which is di- or trisubstituted by hydroxyl or methoxy, or branched or straight-chain butyl which is mono-, di- or trisubstituted by hydroxyl or methoxy, or represents unsubstituted tert.-butyl, or furthermore represents 2-methylthioethyl, trifluoromethylthiomethyl, 2-trifluoromethylthioethyl, cycloalkyl with 3 to 6 carbon atoms, cycloalkenyl which has 5 or 6 carbon atoms and is optionally substituted by hydroxyl, 1,1-dioxidotetrahydrothiophen-3-yl, cyclopropylmethyl, 1-phenethyl or furylmethyl, or represents allyl or propargyl which is optionally substituted by phenyl,
and pharmaceutically usable hydrates, acid addition salts and alkali metal, alkaline earth metal, silver and guanidinium salts thereof, and their esters and other customary prodrug forms.

10. Use of 1-cyclopropyl-6-fluoro-1,4-dihydro-4-oxo-7-(1-piperazinyl)-3-quinolinecarboxylic acids of the formula (I)

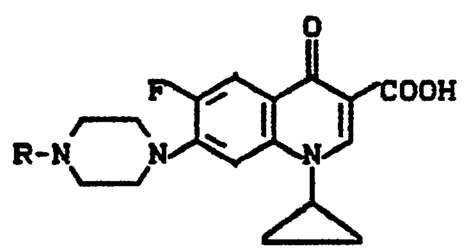

(I)

in which
R represents branched or straight-chain propyl which is di- or trisubstituted by hydroxyl or methoxy, or branched or straight-chain butyl which is mono-, di- or trisubstituted by hydroxyl or methoxy, or represents unsubstituted tert.-butyl, or furthermore represents 2-methylthioethyl, trifluoromethylthiomethyl, 2-trifluoromethylthioethyl, cycloalkyl with 3 to 6 carbon atoms, cycloalkenyl which has 5 or 6 carbon atoms and is optionally substituted by hydroxyl, 1,1-dioxidotetrahydrothiophen-3-yl, cyclopropylmethyl, 1-phenethyl or furylmethyl, or represents allyl or propargyl which is optionally substituted by phenyl,
and pharmaceutically usable hydrates, acid addition salts and alkali metal, alkaline earth metal, silver and guanidinium salts thereof, and their esters and other customary prodrug forms, for the preparation of medicaments.

**Revendications**

1. Acides 1-cyclopropyl-6-fluoro-1,4-dihydro-4-oxo-7-(1-pipérazinyl)-3-quinoléine-carboxyliques de formule (I)

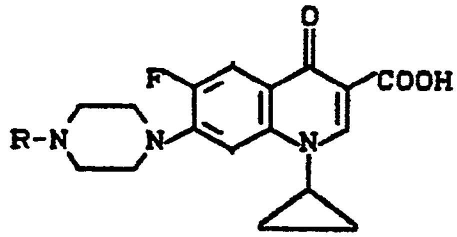

(I)

dans laquelle
R désigne un groupe propyle ramifié ou non ramifié portant deux ou trois substituants hydroxy ou méthoxy, un groupe butyle ramifié ou non ramifié portant 1 à 3 substituants hydroxy ou méthoxy et un groupe tertio-butyle non substitué, en outre, un groupe 2-méthylthioéthyle, trifluorométhylthiométhyle, 2-trifluorométhylthioéthyle, cycloalkyle ayant 3 à 6 atomes de carbone, cycloalcényle de 5 ou 6 atomes de carbone éventuellement substitué par un radical hydroxy, 1,1-dioxydotétrahydrothiophène-3-yle, cyclopropylméthyle, 1-phényléthyle, furylméthyle ainsi qu'un groupe allyle éventuellement substitué par un radical phényle et un groupe propargyle,
et leurs hydrates, leurs sels d'addition d'acides, leurs sels de métaux alcalins, de métaux alcalino-terreux, d'argent et de guanidinium acceptables du point de vue pharmaceutique, ainsi que leurs esters et autres formes classiques de promédicaments.

2. Acides 1-cyclopropyl-6-fluoro-1,4-dihydro-4-oxo-7-(1-pipérazinyl)-3-quinoléine-carboxyliques de formule (I)

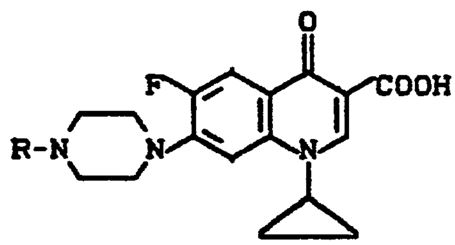

(I)

dans laquelle
R est un groupe propyle ramifié ou non ramifié portant deux ou trois substituants hydroxy ou méthoxy, un groupe butyle ramifié ou non ramifié portant 1 à 3 substituants hydroxy ou méthoxy et un groupe tertio-butyle non substitué, en outre un groupe trifluorométhylthiométhyle, 2-trifluorométhylthioéthyle, cycloalkyle de 3 à 6 atomes de carbone, cyclohexényle éventuellement substitué par un radical hydroxy, 1,1-dioxydotétrahydrothiophène-3-yle, cyclopropylméthyle, furylméthyle ainsi qu'un groupe allyle éventuellement substitué par un radical phényle et un groupe propargyle,
et leurs hydrates, leurs sels d'addition d'acides, leurs sels de métaux alcalins, de métaux alcalino-terreux, d'argent et de guanidinium acceptables du point de vue pharmaceutique, ainsi que leurs esters et autres formes classiques de promédicaments.

3. Procédé de production d'acides 1-cyclopropyl-6-fluoro-1,4-dihydro-4-oxo-7-(1-pipérazinyl)-3-quinoléine-carboxyliques de formule (I)

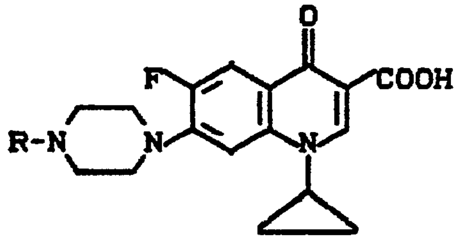

(I)

dans laquelle
R désigne un groupe propyle ramifié ou non ramifié portant deux ou trois substituants hydroxy ou méthoxy, un groupe butyle ramifié ou non ramifié portant 1 à 3 substituants hydroxy ou méthoxy et un groupe tertio-butyle non substitué, en outre, un groupe 2-méthylthioéthyle, trifluorométhylthiométhyle, 2-trifluorométhylthioéthyle, cycloalkyle ayant 3 à 6 atomes de carbone, cycloalcényle de 5 ou 6 atomes de carbone éventuellement substitué par un radical hydroxy, 1,1-dioxydotétrahydrothiophène-3-yle, cyclopropylméthyle, 1-phényléthyle, furylméthyle ainsi qu'un groupe allyle éventuellement substitué par un radical phényle et un groupe propargyle, caractérisé en ce qu'on fait réagir des composés de formule (II)

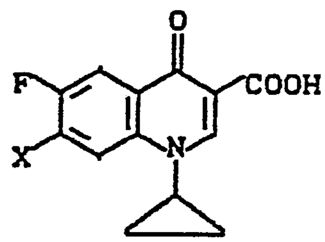

(II),

dans laquelle
X est le fluor ou le chlore,
avec des dérivés de pipérazine de formule (II)

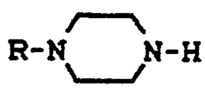

(III)

dans laquelle
R a la définition indiquée ci-dessus, le cas échéant en présence d'accepteurs d'acides.

4. Procédé suivant la revendication 3, caractérisé en ce qu'on utilise 1 à 15 moles de la pipérazine (III) par mole de l'acide quinolone-carboxylique (II).

5. Procédé de production d'acides 1-cyclopropyl-6-fluoro-1,4-dihydro-4-oxo-7-(1-pipérazinyl)-3-quinoléine-carboxyliques de formule (I)

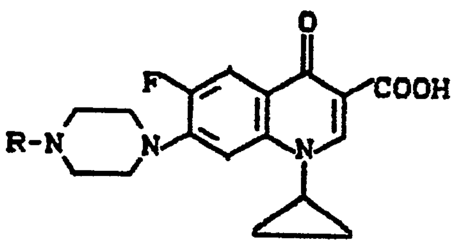

(I)

dans laquelle
R désigne un groupe propyle ramifié ou non ramifié portant deux ou trois substituants hydroxy ou méthoxy, un groupe butyle ramifié ou non ramifié portant 1 à 3 substituants hydroxy ou méthoxy et un groupe tertio-butyle non substitué, en outre, un groupe 2-méthylthioéthyle, trifluorométhylthiométhyle, 2-trifluorométhylthioéthyle, cycloalkyle ayant 3 à 6 atomes de carbone, cycloalcényle de 5 ou 6 atomes de carbone éventuellement substitué par un radical hydroxy, 1,1-dioxydotétrahydrothiophène-3-yle, cyclopropylméthyle, 1-phényléthyle, furylméthyle ainsi qu'un groupe allyle éventuellement substitué par un radical phényle et un groupe propargyle, caractérisé en ce qu'on fait réagir l'acide 1-cyclopropyl-6-fluoro-1,4-dihydro-4-oxo-7-(1-pipérazinyl)-3-quinoléine-carboxylique de formule (IV)

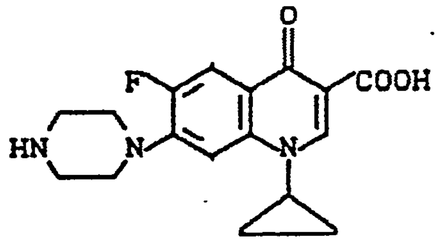

(IV)

avec des composés de formule (V)

R–Y (V),

dans laquelle
R a la définition indiquée ci-dessus, mais ne peut pas être un groupe tertio-butyle, tris(hydroxyméthyl)-méthyle ou cycloalkyle, et
Y est un halogène, notamment le chlore, le brome ou l'iode,
éventuellement en présence d'accepteurs d'acides.

6. Procédé suivant la revendication 5, caractérisé en ce qu'on utilise 1 à 4 moles du composé (V) par mole du composé (IV).

7. Procédé suivant la revendication 5, caractérisé en ce qu'il est mis en œuvre en présence de catalyseurs de transfert de phase dans un diluant.

8. Acides 1-cyclopropyl-6-fluoro-1,4-dihydro-4-oxo-7-(1-pipérazinyl)-3-quinoléine-carboxyliques de formule (I)

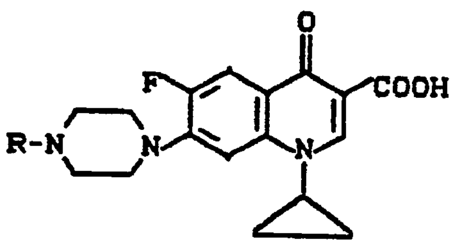

(I)

dans laquelle
R désigne un groupe propyle ramifié ou non ramifié portant deux ou trois substituants hydroxy ou méthoxy, un groupe butyle ramifié ou non ramifié portant 1 à 3 substituants hydroxy ou méthoxy et un groupe tertio-butyle non substitué, en outre, un groupe 2-méthylthioéthyle, trifluorométhylthiométhyle, 2-trifluorométhylthioéthyle, cycloalkyle ayant 3 à 6 atomes de carbone, cycloalcényle de 5 ou 6 atomes de carbone éventuellement substitué par un radical hydroxy, 1,1-dioxydotétrahydrothiophène-3-yle, cyclopropylméthyle, 1-phényléthyle, furylméthyle ainsi qu'un groupe allyle éventuellement substitué par un radical phényle et un groupe propargyle,
et leurs hydrates, leurs sels d'addition d'acides, leurs sels de métaux alcalins, de métaux alcalino-terreux, d'argent et de guanidinium acceptables du point de vue pharmaceutique, ainsi que leurs esters et leurs formes classiques de promédicaments, destinés à être utilisés dans un procédé de traitement thérapeutique du corps humain ou animal.

9. Médicament contenant des acides 1-cyclopropyl-6-fluoro-1,4-dihydro-4-oxo-7-(1-pipérazinyl)-3-quinoléine-carboxyliques de formule (I)

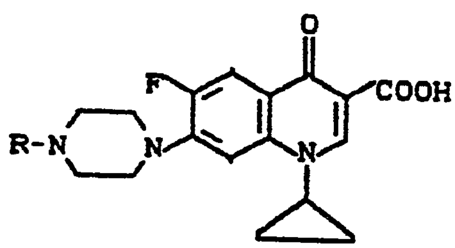

(I)

dans laquelle
R désigne un groupe propyle ramifié ou non ramifié portant deux ou trois substituants hydroxy ou méthoxy, un groupe butyle ramifié ou non ramifié portant 1 à 3 substituants hydroxy ou méthoxy et un groupe tertio-butyle non substitué, en outre, un groupe 2-méthylthioéthyle, trifluorométhylthiométhyle, 2-trifluorométhylthioéthyle, cycloalkyle ayant 3 à 6 atomes de carbone, cycloalcényle de 5 ou 6 atomes de carbone éventuellement substitué par un radical hydroxy, 1,1-dioxydotétrahydrothiophène-3-yle, cyclopropylméthyle, 1-phényléthyle, furylméthyle ainsi qu'un groupe allyle éventuellement substitué par un radical phényle et un groupe propargyle,

et leurs hydrates, leurs sels d'addition d'acides, leurs sels de métaux alcalins, de métaux alcalino-terreux, d'argent et de guanidinium acceptables du point de vue pharmaceutique, de même que leurs esters et autres formes classiques de promédicaments.

10. Utilisation d'acides 1-cyclopropyl-6-fluoro-1,4-dihydro-4-oxo-7-(1-pipérazinyl)-3-quinoléine-carboxyliques de formule (I)

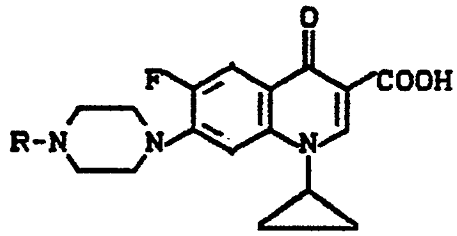

(I)

dans laquelle

R désigne un groupe propyle ramifié ou non ramifié portant deux ou trois substituants hydroxy ou méthoxy, un groupe butyle ramifié ou non ramifié portant 1 à 3 substituants hydroxy ou méthoxy et un groupe tertio-butyle non substitué, en outre, un groupe 2-méthylthioéthyle, trifluorométhylthiométhyle, 2-trifluorométhylthioéthyle, cycloalkyle ayant 3 à 6 atomes de carbone, cycloalcényle de 5 ou 6 atomes de carbone éventuellement substitué par un radical hydroxy, 1,1-dioxydotétrahydrothiophène-3-yle, cyclopropylméthyle, 1-phényléthyle, furylméthyle ainsi qu'un groupe allyle éventuellement substitué par un radical phényle et un groupe propargyle,

et de leurs hydrates, leurs sels d'addition d'acides, leurs sels de métaux alcalins, de métaux alcalino-terreux, d'argent et de guanidinium, de même que de leurs esters et autres formes classiques de promédicaments pour la préparation de médicaments.